# FASCICULE DE BREVET EUROPEEN

(11) **EP 1 654 016 B1**
(45) Date de publication et mention de la délivrance du brevet: **27.03.2013**
(21) Numéro de dépôt: 04767387.6
(22) Date de dépôt: 18.06.2004
(51) Int. Cl.: A61L 31/16, A61L 31/10

(54) **PROTHESES AVEC REVETEMENTS BIOLOGIQUEMENT ACTIFS**
PROTHESEN MIT BIOLOGISCH AKTIVEN BESCHICHTUNGEN
PROSTHESES WITH BIOLOGICALLY ACTIVE COVERINGS

(30) Priorité: 19.06.2003 FR 0307395
(43) Date de publication de la demande: 10.05.2006
(73) Titulaire: Universite Paris 5, 75270 Paris Cedex 06 (FR)
(72) Inventeur: LAFONT, Antoine, F-75007 Paris (FR); AL HAJ ZEN, Ayman, F-92130 Issy les Moulineaux (FR)
(74) Mandataire: Cornet, Nicolas Yves Pascal
(86) Numéro de dépôt international: PCT/FR2004/001528
(87) Numéro de publication internationale: WO 2004/112861

(56) Documents cités:
- WO-A1-00/40278
- WO-A1-01/43789
- US-A- 5 650 389
- US-B1- 6 579 978
- FISCHER JENS W ET AL: "Local expression of bovine decorin by cell-mediated gene transfer reduces neointimal formation after balloon injury in rats" CIRCULATION RESEARCH, vol. 86, no. 6, 31 mars 2000 (2000-03-31), pages 676-683, XP002315415 ISSN: 0009-7330

## Description

L'invention a pour objet l'utilisation de décorine et/ou fragment peptidique de la décorine et/ou dérivé chimiquement modifié de décorine pour le revêtement de stents ou prothèses endovasculaires. Elle vise en particulier le revêtement des stents comportant un revêtement biologiquement actif.

On sait que le traitement des sténoses des artères coronaires a été révolutionné par l'angioplastie coronaire, qui consiste à ouvrir la sténose avec un ballonnet. Cette technique a été perfectionnée par le déploiement d'une endoprothèse artérielle en métal, appelée « stent », afin d'éviter la cicatrisation rétractile de l'artère entraînant la resténose, c -à -d la réapparition de la sténose. Cependant, dans un bon nombre de cas, variant de 20 à 40% selon le type de lésion, on a constaté que la mise en place d'un stent dans une artère entraîne une resténose liée à une hyperplasie néo-intimale, qui résulte à la fois d'un excès de tissu cicatriciel et d'une réaction au corps étranger. Pour surmonter ces problèmes, on a proposé de recouvrir les stents de substances médicamenteuses capables de lutter contre les resténoses.

Parmi les stratégies proposées, celle consistant à utiliser des molécules à effet cytotoxiques ou cytostatiques a suscité un grand intérêt. En effet, sur les 6- premiers mois de la mise en place de stents avec revêtement de composés cytotoxiques ou cytostatiques, aucune resténose n'a été observée.

Toutefois, ces molécules présentent l'inconvénient d'inhiber également la phase cicatricielle,ce qui entraîne un risque de thrombose tardive sur un corps métallique nu, ainsi que la création d'un espace entre le stent et la paroi artérielle par dilatation de cette paroi (désigné ci-après par remodelage positif).

Sur des modèles animaux, on a également observé un phénomène tardif de resténose.

Il s'avère donc que si l'utilisation des stents en tant que plate-formes pharmacologiques permettant de délivrer un médicament, constitue une approche d'intérêt, les familles thérapeutiques proposées à ce jour ne sont pas satisfaisantes.

Les inventeurs ont constaté qu'en suivant une autre approche médicamenteuse, reposant sur la mise à profit de composés multifonctionnels, il était possible d'exercer un effet de régulation sur la matrice extracellulaire, et d'inhiber le tissu cicatriciel responsable de l'hyperplasie, empêchant ainsi la resténose intra-stent. Ce résultat s'est avéré généralisable au revêtement d'autres prothèses dans d'autres indications médicales, et de manière générale à toute plate-forme biologique.

L'invention repose donc sur une stratégie à effets multiples visant la prolifération et de la migration cellulaire, le métabolisme de la matrice extracellulaire et le contrôle de l'inflammation.

L'invention a donc pour but d'utiliser décorine et/ou fragment peptidique de la décorine et/ou dérivé chimiquement modifié de décorine dans l'élaboration de revêtements de stents ou prothèses endovasculaires.

Elle vise également, en tant que nouveaux produits, ces stents ou prothèses endovasculaires portant de tels revêtements.

L'utilisation selon l'invention est caractérisée par la mise en oeuvre de décorine et/ou fragment peptidique de la décorine et/ou dérivé chimiquement modifié de décorine pour élaborer un revêtement pharmacologiquement actif sur un stent ou une prothèse endovasculaire.

De manière inattendue, de tels revêtements permettent, dans une situation de traumatisme mécanique des tissus provoquant une réaction inflammatoire de prévenir la resténose artérielle.

Contrairement aux stratégies de l'art antérieur évoquées ci-dessus, de tels régulateurs n'interviennent pas sur le cycle cellulaire et n'entraînent donc pas d'action délétère sur l'endothélium pouvant résulter dans l'apparition de thromboses tardives, un remodelage positif ou une resténose tardive.

Il est ainsi possible de maintenir une paroi saine et non altérée par la perte ou l'endommagement de cellules, ce qui permet aussi de prévenir les phénomènes de thromboses.

De manière préférée, on utilise la décorine et/ou un fragment peptidique de la décorine, ou les dérivés de décorine et/ ou de fragment de décorine, possédant les propriétés de ces composés, mais chimiquement modifiés pour leur conférer des propriétés avantageuses pour une application donnée.

La décorine humaine est une protéine de 359 acides aminés avec une chaîne de glycosaminoglycans, de PM de 100 à 120 kDa. Elle correspond à la séquence suivante:
mkatiillll aqvswagpfq qrglfdfmle deasgigpev pddrdfepsl gpvcpfrcqc hlrvvqcsdl gldkvpkdlp pdttlldlqn nkiteikdgd fknlknlhal ilvnnkiskv spgaftplvk lerlylsknq lkelpekmpk tlqelrahen eitkvrkvtf nglnqmivie lgtnplkssg iengafqgmk klsyiriadt nitsipqglp psltelhldg nkisrvdaas Ikglnnlakl glsfnsisav dngslantph lrelhldnnk ltrvpgglae hkyiqwylh nnnisvvgss dfcppghntk kasysgvslf snpvqyweiq pstfrcvyvr saiqlgnyk

La décorine utilisée selon l'invention correspond avantageusement aux domaines suivants :
- Domaine I : Signal peptide + propeptide,
- Domaine II : Résidus de la cystéine + site d'attachement des glycosaminoglycanes (GAGs)
- Domaine III : Répétitions riches en leucine (LRR), coeur protéique (38-43 kDa),
- Domaine IV : Résidus de la cystéine avec boucle.

Le fragment actif protéique proposé alternativement est défini comme suit : fragment de décorine bioactive entre l'acide aminé en positions (115) et (260), 15-20 kDa,

La présence de ces composés sur un stent ou une prothèse endovasculaire permet de mettre à profit leurs propriétés multifonctionnelles. Il est ainsi possible d'agir sur la prolifération cellulaire (en inhibant l'action du PDGF et de l'EGF, en se fixant sur le récepteur de l'EGF), sur la migration cellulaire (en inhibant la migration par action sur la fibronectine et la trombospondine, et en inhibant la dégradation de la matrice extracellulaire), sur l'inflammation (en réduisant l'infiltration des macrophages ; en inhibant l'action inflammatoire de l'interleukine 1 et la réaction inflammatoire au traumatisme de l'angioplastie sur les cellules musculaires lisses par maintien de leur phénotype contractile (non sécrétoire de matrice extracellulaire et de cytokines pro-inflammatoires)), et en agissant contre la fibrose (par inhibition de l'accumulation de la matrice extracellulaire, notamment par l'intermédiaire de son action sur l'interleukine 1, le TGFβ-1 et le PDGF BB).

Selon un autre aspect, l'invention vise également, en tant que nouveaux produits, des stents ou prothèses endovasculaires caractérisées en ce qu'elles comportent un revêtement comprenant une quantité thérapeutiquement efficace décorine et/ou fragment peptidique de la décorine et/ou dérivé chimiquement modifié de décorine.

Par « quantité thérapeutiquement efficace », on entend une quantité qui permet d'obtenir les effets rapportés ci-dessus, en particulier de réguler, notamment d'inhiber le surplus de matrice extracellulaire produit en réaction au traumatisme de stent ou de la prothèse endovasculaire mise en place. Des quantités de l'ordre de 10 à 100 µg/mm² se sont révélées appropriées.

Ces composés sont fixées directement au stent ou à la prothèse endovasculaire, ou par l'intermédiaire d'un revêtement biostable ou biodégradable comme un polymère d'acide lactique. La liaison des composés peut être réversible ou irréversible. Les stents prothèses endovasculaires peuvent être biodégradables, par exemple en polymère d'acide lactique. Elles peuvent être aussi en manganèse. La libération peut soit ne pas avoir lieu, soit avoir lieu avec une vitesse qui dépend du revêtement, de la liaison utilisée, du stent ou de la prothèse endovasculaire (dégradable ou non).

L'effet anti-fibrosant de la décorine et d'un fragment de décorine est avantageusement mis à profit également sur des prothèses hors de l'application artérielle, notamment dans des applications urologique, digestive, broncho-pulmonaire.

Dans ces applications, les composés utilisés sont fixés sur le stent ou la prothèse endovasculaire par exemple métallique, ou biorésorbable. Cette fixation peut être transitoire ou définitive. Le composé agit alors dans la proximité du stent ou de la prothèse endovasculaire, cette zone étant à l'origine du déclenchement de l'inflammation la plus importante et donc de la prolifération et de la migration cellulaire, et de l'accumulation de matrice extracellulaire.

D'autres caractéristiques et avantages de l'invention sont donnés dans les exemples qui suivent.

### Réalisation de stents avec revêtement bioactif de décorine et application artérielle

En opérant selon les techniques classiques, on applique sur un stent métallique par exemple en acier 316L un revêtement biostable ou biodégradable à base de polymères, par exemple un polymère d'acide lactique, comportant une quantité pharmacologiquement active de décorine, permettant la libération de principe actif sur 30 jours.

*In vivo,* la décorine inhibe localement la resténose au niveau de l'artère iliaque de lapin. Après 2 mois d'observations, on n'a observé aucun phénomène de resténose.

En variante, la décorine est fixée directement au stent sans revêtement.

## Revendications

1. Décorine et/ou fragment peptidique de la décorine et/ou dérivé chimiquement modifié de décorine pour son utilisation, en tant que revêtement pharmacologiquement actif sur un stent ou une prothèse endovasculaire, pour la prévention et/ou le traitement de la resténose.

2. Décorine et/ou fragment peptidique de la décorine et/ou dérivé chimiquement modifié de décorine pour son utilisation selon la revendication 1, dans laquelle ladite décorine consiste en la SEQ ID NO :1.

3. Décorine et/ou fragment peptidique de la décorine et/ou dérivé chimiquement modifié de décorine pour son utilisation selon la revendication 1 ou 2, dans laquelle ledit fragment de la décorine comprend les acides aminés 115 à 260 de la SEQ ID NO :1.

4. Stent ou prothèse endovasculaire, **caractérisés en ce qu'**il comporte un revêtement comprenant une quantité thérapeutiquement efficace de décorine et/ou de fragment peptidique de la décorine et/ou d'un dérivé chimiquement modifié de décorine.

5. Stent ou prothèse endovasculaire selon la revendication 4, dans lequel ledit stent, ou ladite prothèse endovasculaire est constitué d'un polymère d'acide lactique.

6. Stent ou prothèse endovasculaire selon la revendication 4 ou 5, dans lequel la décorine consiste en la SEQ ID NO :1.

7. Stent ou prothèse endovasculaire selon l'une quelconque des revendications 4 à 6, dans lequel le fragment de la décorine comprend les acides aminés 115 à 260 de la SEQ ID NO :1.

8. Stent ou prothèse endovasculaire selon l'une quelconque des revendications 4 à 7, dans lequel la quantité thérapeutiquement efficace de décorine et/ou de fragment peptidique de la décorine et/ou d'un dérivé chimiquement modifié de décorine est de 10 à 100µg/mm³.

9. Stent ou prothèse endovasculaire selon l'une quelconque des revendications 4 à 8, dans lequel ledit revêtement est biostable ou biodégradable.

## Claims

1. Decorin and/or decorin peptide fragment and/or chemically modified decorin derivative for its use, as a pharmacologically active coating on a stent or an endovascular prosthesis, for the prevention and/or treatment of restenosis.

2. Decorin and/or decorin peptide fragment and/or chemically modified decorin derivative for its use according to claim 1, wherein said decorin consists of SEQ ID NO: 1.

3. Decorin and/or decorin peptide fragment and/or chemically modified decorin derivative for its use according to claim 1 or 2, wherein said decorin fragment comprises amino acids 115 to 260 of SEQ ID NO: 1.

4. Stent or endovascular prosthesis, **characterised in that** it has a coating comprising a therapeutically effective quantity of decorin and/or of a decorin peptide fragment and/or of a chemically modified decorin derivative.

5. Stent or endovascular prosthesis according to claim 4, wherein said stent or said endovascular prosthesis is composed of a lactic acid polymer.

6. Stent or endovascular prosthesis according to claim 4 or 5, wherein the decorin consists of SEQ ID NO: 1.

7. Stent or endovascular prosthesis according to any one of claims 4 to 6, wherein the decorin fragment comprises amino acids 115 to 260 of SEQ ID NO: 1.

8. Stent or endovascular prosthesis according to any one of claims 4 to 7, wherein the therapeutically effective quantity of decorin and/or of a decorin peptide fragment and/or of a chemically modified decorin derivative is from 10 to 100 µg/mm³.

9. Stent or endovascular prosthesis according to any one of claims 4 to 8, wherein said coating is biostable or biodegradable.

## Patentansprüche

1. Decorin und/oder Peptidfragment von Decorin und/oder chemisch modifiziertes Derivat von Decorin für seine Verwendung als pharmazeutisch aktive Beschichtung auf einem Stent oder einer intravaskulären Prothese zur Verhütung und/oder Behandlung von Restenose.

2. Decorin und/oder Peptidfragment von Decorin und/oder chemisch modifiziertes Derivat von Decorin für seine Verwendung nach Patentanspruch 1, in der das genannte Decorin aus der SEQ ID NO : 1 besteht.

3. Decorin und/oder Peptidfragment von Decorin und/oder chemisch modifiziertes Derivat von Decorin für seine Verwendung nach Patentanspruch 1 oder 2, in der das genannte Decorinfragment die Aminosäuren 115 bis 260 der SEQ ID NO : 1 umfasst.

4. Stent oder intravaskuläre Prothese, **dadurch gekennzeichnet, dass** es eine Beschichtung aufweist, die eine therapeutisch wirksame Menge von Decorin und/oder einem Peptidfragment von Decorin und/oder eines chemisch modifizierten Derivats von Decorin umfasst.

5. Stent oder intravaskuläre Prothese nach Patentanspruch 4, wobei der genannte Stent oder die genannte intravaskuläre Prothese aus einem Milchsäurepolymer besteht.

6. Stent oder intravaskuläre Prothese nach Patentanspruch 4 oder 5, indem das Decorin aus der SEQ ID NO : 1 besteht.

7. Stent oder intravaskuläre Prothese nach irgendeinem der Patentansprüche 4 bis 6, in dem das Decorinfragment die Aminosäuren 115 bis 260 der SEQ ID NO : 1 umfasst.

8. Stent oder intravaskuläre Prothese nach irgendeinem der Patentansprüche 4 bis 7, in dem die therapeutisch wirksame Menge von Decorin und/oder einem Peptidfragment von Decorin und/oder eines chemisch modifizierten Derivats von Decorin 10 bis 100 µg/mm³ beträgt.

9. Stent oder intravaskuläre Prothese nach irgendeinem der Patentansprüche 4 bis 8, in dem die genannte Beschichtung biologisch stabil oder biologisch abbaubar ist.


### SEQUENCE LISTING

<110> Institut National de la Santé et de la Recherche Médicale (INSERM)
   UNIVERSITE PARIS 5
<120> "PLATE-FORMES, NOTAMMENT PROTHESES, AVEC REVETEMENTS BIOLOGIQUEMENT ACTIFS"
<130> CP/BB 61017-2067
<150> FR 03 07 395
   <151> 2003-06-19
<160> 1
<170> PatentIn version 3.1
<210> 1
   <211> 359
   <212> PRT
   <213> Human
<400> 1
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Gly Leu Asp Lys Val Pro Lys Asp Leu Pro
   65                  70                  75                  80
   Pro Asp Thr Thr Leu Leu Asp Leu Gln Asn Asn Lys Ile Thr Glu Ile
                   85                  90                  95
   Lys Asp Gly Asp Phe Lys Asn Leu Lys Asn Leu His Ala Leu Ile Leu
               100                 105                 110
   Val Asn Asn Lys Ile Ser Lys Val Ser Pro Gly Ala Phe Thr Pro Leu
           115                 120                 125
   Val Lys Leu Glu Arg Leu Tyr Leu Ser Lys Asn Gln Leu Lys Glu Leu
       130                 135                 140
   Pro Glu Lys Met Pro Lys Thr Leu Gln Glu Leu Arg Ala His Glu Asn
   145                 150                 155                 160
   Glu Ile Thr Lys Val Arg Lys Val Thr Phe Asn Gly Leu Asn Gln Met
                   165                 170                 175
   Ile Val Ile Glu Leu Gly Thr Asn Pro Leu Lys Ser Ser Gly Ile Glu
               180                 185                 190
   Asn Gly Ala Phe Gln Gly Met Lys Lys Leu Ser Tyr Ile Arg Ile Ala
           195                 200                 205
   Asp Thr Asn Ile Thr Ser Ile Pro Gln Gly Leu Pro Pro Ser Leu Thr
       210                 215                 220
   Glu Leu His Leu Asp Gly Asn Lys Ile Ser Arg Val Asp Ala Ala Ser
   225                 230                 235                 240
   Leu Lys Gly Leu Asn Asn Leu Ala Lys Leu Gly Leu Ser Phe Asn Ser
                   245                 250                 255
   Ile Ser Ala Val Asp Asn Gly Ser Leu Ala Asn Thr Pro His Leu Arg
               260                 265                 270
   Glu Leu His Leu Asp Asn Asn Lys Leu Thr Arg Val Pro Gly Gly Leu
           275                 280                 285
   Ala Glu His Lys Tyr Ile Gln Val Val Tyr Leu His Asn Asn Asn Ile
       290                 295                 300
   Ser Val Val Gly Ser Ser Asp Phe Cys Pro Pro Gly His Asn Thr Lys
   305                 310                 315                 320
   Lys Ala Ser Tyr Ser Gly Val Ser Leu Phe Ser Asn Pro Val Gln Tyr
                   325                 330                 335
   Trp Glu Ile Gln Pro Ser Thr Phe Arg Cys Val Tyr Val Arg Ser Ala
               340                 345                 350
   Ile Gln Leu Gly Asn Tyr Lys
           355